# EUROPÄISCHE PATENTSCHRIFT

(11) **EP 1 721 613 B1**
(45) Veröffentlichungstag und Bekanntmachung des Hinweises auf die Patenterteilung: **28.11.2007**
(21) Anmeldenummer: 05010417.3
(22) Anmeldetag: 13.05.2005
(51) Int. Cl.: A61K 31/7016, A61P 25/08

(54) **Hesperidin zur Behandlung von Epilepsie**
Hesperidin for the treatment of epilepsy
Hespéridine pour le traitement de l'épilepsie

(43) Veröffentlichungstag der Anmeldung: 15.11.2006
(73) Patentinhaber: Mewicon med. wiss. Beratung GmbH, 4164 Schwarzenberg (AT)
(72) Erfinder: Dimpfel, Wilfried Prof. Dr., 35415 Pohlheim (DE)
(74) Vertreter: Maiwald Patentanwalts GmbH

(56) Entgegenhaltungen:
- WO-A-01/30331
- WO-A-02/066032
- WO-A-03/061678
- FR-A- 2 319 361
- US-A- 4 496 548
- US-B1- 6 232 294
- SUAREZ J ET AL: "Effect of hesperidin and neohesperidin dihydrochalcone on central nervous system" FITOTERAPIA, Bd. 67, Nr. 4, 1996, Seiten 359-363, XP008056056 ISSN: 0367-326X
- GARG A ET AL: "Chemistry and pharmacology of the Citrus bioflavonoid hesperidin" PHYTOTHERAPY RESEARCH 2001 UNITED KINGDOM, Bd. 15, Nr. 8, 2001, Seiten 655-669, XP008038270 ISSN: 0951-418X
- DATABASE WPI Section Ch, Week 200403 Derwent Publications Ltd., London, GB; Class B04, AN 2004-027380 XP002304172 & JP 2003 325135 A (TOYO SHINYAKU KK) 18. November 2003 (2003-11-18)
- KIM, JI YOUNG ET AL: "Hesperetin: A potent antioxidant against peroxynitrite" FREE RADICAL RESEARCH , 38(7), 761-769 CODEN: FRARER; ISSN: 1071-5762, 2004, XP008038013

## Beschreibung

Die vorliegende Erfindung betrifft die Verwendung von Hesperidin (bzw. als Aglykon Hesperetin) oder seinen pharmazeutisch annehmbaren Salzen zur Prophylaxe und Behandlung von Epilepsie.

### Hintergrund der Erfindung

Hesperidin, auch als Hesperetin 7-rhamnoglucosid bekannt, hat folgende Summenformel: C28H34015 und die allgemeine Formel:

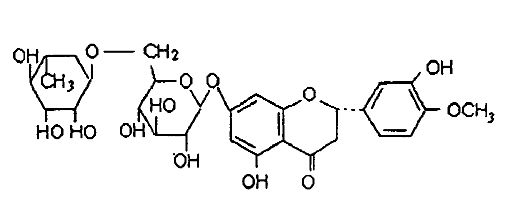

Hesperidin (sein Aglykon Hesperetin entsteht im Darm durch die Tätigkeit von Bakterien) ist ein pflanzlicher Inhaltsstoff, der vorwiegend in Zitrusfrüchten (Orangen) vorkommt. Hesperidin wird auch als Bestandteil von Kombinationspräparaten (Cyclo 3 fort, Daflon) zur Behandlung venöser Gefäßerkrankungen verwendet. Chemie und bislang bekannte Wirkungen von Hesperidin wurden von Garg et al., 2001 (Garg A, Garg S, Zaneveld LJD, Singla AK (2001) Chemistry and Pharmacology of the Citrus Bioflavonoid Hesperidin. Phytother. Res. 15: 655-669) umfassend beschrieben. Auf der Basis der bisher bekannt gewordenen Untersuchungen war eine Wirkung von Hesperidin bei Epilepsie nicht zu erwarten gewesen. Erst die Anwendung der völlig "bias"-freien Untersuchung im Tele-Stereo-EEG der Ratte erbrachte im Rahmen von Versuchen mit anderer Zielsetzung den Hinweis auf das Vorhandensein einer derartigen zentralnervösen Wirkung. Die Wirkung wurde weiterhin durch in vitro Versuche am Hippokampus-Schnittpräparat in direkter Interaktion zwischen Wirkstoff und seinem Zielorgan, dem Gehirn, untermauert. Insgesamt ergaben die Versuche ein unterschiedliches Wirkprofil für Hesperidin und Hesperetin.

Die derzeit üblichen Arzneimittel zur Behandlung von Erregbarkeitsstörungen des zentralen Nervensystems, insbesondere von Epilepsie haben ein breites Nebenwirkungsspektrum, insbesondere hinsichtlich des Auftretens von Müdigkeit. Nahezu alle bislang verfügbaren Medikamente beeinträchtigen die Fahrtüchtigkeit. Sehr viele Formen der Epilepsie sind medikamentös bislang überhaupt nicht oder nur äußerst schwer mit Hilfe von Medikamenten zu beherrschen. Oft bleibt nur eine chirurgische Alternative mit den bekannten Risiken. Es besteht daher ein sehr großer Bedarf nach einer Prophylaxe bzw. einem verbesserten Arzneimittel mit einer guten therapeutischen Wirksamkeit bei möglichst geringer Nebenwirkungsrate. Insbesondere gilt dies auch für die Prophylaxe, z.B. für die Verabreichung von Substanzen als Nahrungsergänzungsmittel. Dies ist bei Verabreichung eines Naturstoffes eher zu erwarten.

Diese Aufgabe wurde überraschenderweise durch die Verwendung des Naturstoffs Hesperidin (sowie von dem im Darm entstehenden Aglykon Hesperetin) bzw. deren pharmazeutisch annehmbaren Salzen als Wirkstoff für die Herstellung eines Arzneimittels zur Prophylaxe und Behandlung von Epilepsie gelöst. Hierbei ist die Anwendung sowohl Dosis-abhängig wie auch abhängig von der Applikationsart, da Hesperidin und Hesperetin im unteren Dosisbereich zwar ähnlich wirken, jedoch bei höherer Dosierung ein unterschiedliches Wirkprofil erkennen lassen. Hesperetin entsteht im Darm nach oraler Applikation von Hesperidin durch Tätigkeit der Darmbakterien. Bei oraler Gabe kommen daher beide Substanzen zur Wirkung und ergänzen sich in ihrem Wirkungsprofil. Die überraschend gefundene Wirkung wurde in vivo wie auch in vitro an der Ratte, sowie letztendlich auch in einem Therapieversuch bei einem Patienten mit einer bislang mit Medikamenten kaum behandelbaren Form der Epilepsie, der autosomal vererbbaren, dominanten nocturnen Frontallappenepilepsie bewiesen.

### Zusammenfassung der Erfindung

Die Erfindung betrifft die Verwendung von Hesperidin bzw. seinem Aglykon Hesperetin zur Prophylaxe und Behandlung von Epilepsie.

In vitro wie auch in vivo Versuche an der Ratte zeigen Ergebnisse, wie sie für Medikamente zur Behandlung von Epilepsie typisch sind.

Eine erste Behandlung mit diesen Substanzen bei einer autosomal vererbten nocturnen Frontalllappen-Epilepsie zeigt die vermutete Wirksamkeit.

### Detaillierte Beschreibung der Erfindung

Bei Untersuchungen der Veränderungen der EEG-Frequenzen der Ratte nach Gabe von Hesperidin und Hesperetin sowie der direkten Interaktion von Hesperidin und Hesperetin mit Himmaterie im Hippokampusschnittpräparat der Ratte in vitro wurde überraschenderweise gefunden, dass das Präparat eine Wirkung im Gehirn besitzt, die bei der Behandlung von Epilepsie nützlich ist.

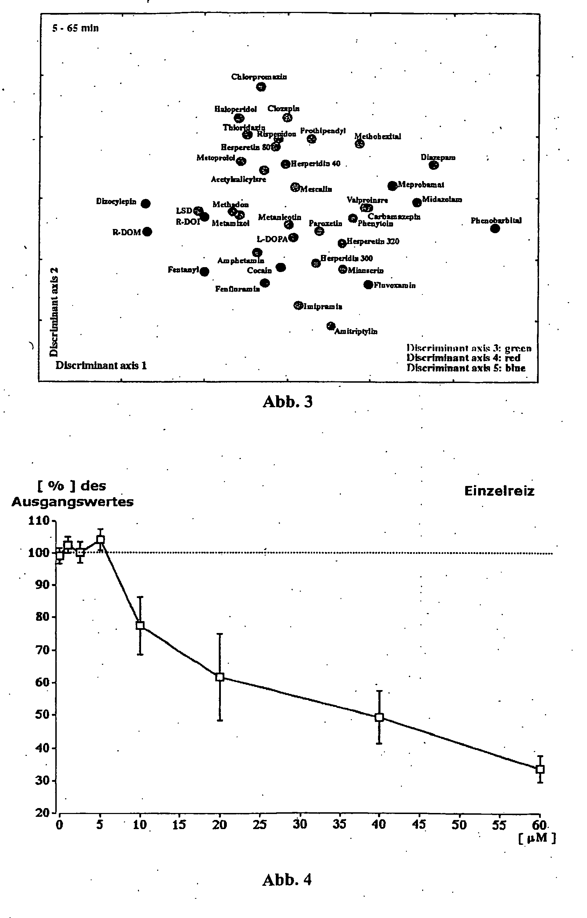

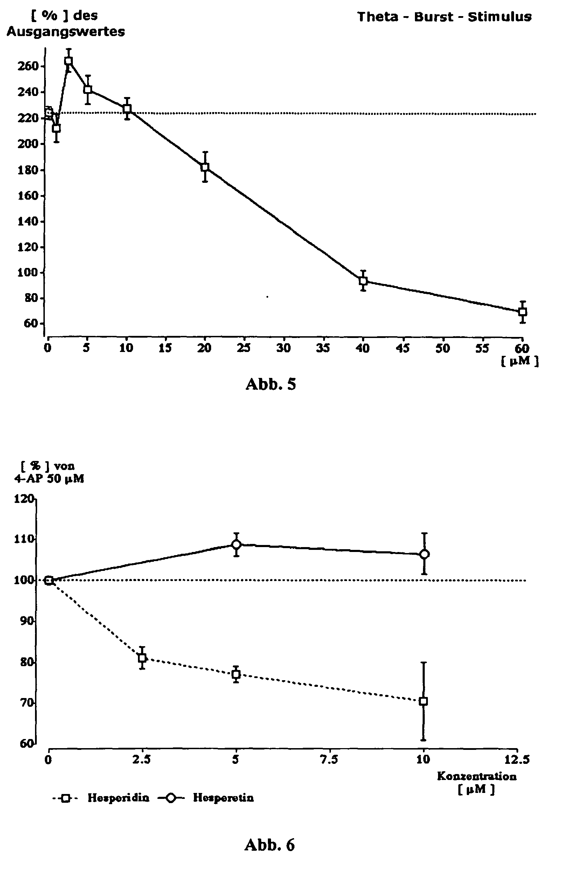

Hesperetin zeigt überraschenderweise im Modell Tele-Stereo-EEG bei Ratten Veränderungen der EEG-Frequenzen, wie sie nach Gabe klassischer Medikamente zur Behandlung von Epilepsie gemessen werden. Die Veränderungen, wie sie nach Gabe klassischer Medikamente sowie nach Gabe von einer hohen Dosis von Hesperetin (320 mg/kg) auftraten, sind gezeigt. Aus der in Abb. 3 gezeigten Diskriminanzanalyse ist offensichtlich, dass sich der charakteristische elektrische Fingerprint, der für klassische Medikamente zur Behandlung von Epilepsie typisch ist, auch in den Mustern von Hesperidin und Hesperetin in signifikanter Weise wiederfindet.
Aus der Erkenntnis, dass Hesperidin und Hesperetin die gleichen charakteristischen Veränderungen der EEG-Frequenzen hervorruft wie üblicherweise Arzneimittel, die zur Behandlung von Epilepsie eingesetzt werden, kann gefolgert werden, dass Hesperidin bzw. Hesperetin bei der Behandlung in denselben Indikationen wirksam ist. Hierbei ist allerdings auch die Dosierung zu berücksichtigen. Dass Medikamente, die in der gleichen Indikation eingesetzt werden, auch gleichartige EEG Veränderungen hervorrufen, konnte von Dimpfel anhand von mehr als 40 Referenzsubstanzen für 8 verschiedene Indikationen gezeigt werden (Dimpfel W Preclinical data base of pharmaco-specific rat EEG fingerprints (Tele-Stereo-EEG). Eur J Med Res (2003) 8: 199-207).
Zusätzlich wurden Untersuchungen am Modell Hippokampus-Schnittpräparat in vitro durchgeführt. In diesen Untersuchungen wurde überraschend gefunden, dass Hesperidin sowohl eine Unterdrückung der Pyramidenzellaktivität nach Einzelreizung wie auch eine Unterdrückung der Langzeitpotenzierung hervorruft (siehe Abb. 5).

Erfindungsgemäß wird Hesperidin (bzw. sein Aglykon Hesperetin) eingesetzt. Diese Substanz, auch als Hesperetin 7-rhamnoglucosid, bekannt, hat folgende Summenformel: C28H34O15 bzw. allgemeine Formel:

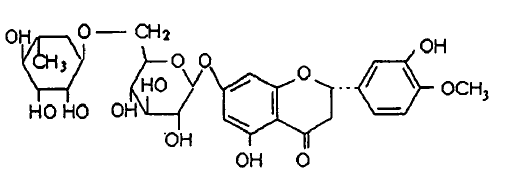

Hesperidin oder Hesperetin kann erfindungsgemäß auch in Form seiner pharmazeutisch annehmbaren Salze eingesetzt werden. Die Herstellung solcher Salze erfolgt in an sich bekannter Weise. Als Salzbildner kommen alle üblichen pharmazeutisch annehmbaren Säuren bzw. Anionen in Frage. Auch eine Kopplung an Glucuronsäure kann verwendet werden. Insbesondere kann auch Hesperidin (Glycosid) ohne seine Zuckerkomponente als Hesperetin (Aglycon) verwendet werden.

Die erfindungsgemäßen Hesperidin oder Hesperetin als Wirkstoff enthaltenden Nahrungsergänzungsmittel bzw. Arzneimittel werden bevorzugt oral verabreicht. Es ist jedoch auch eine Verabreichung auf anderem Wege, z.B. peroral, topisch, parenteral, intravenös, intramuskulär, subkutan, nasal, inhalativ, rektal oder transdermal möglich.

Zur Verabreichung kann das erfindungsgemäße Arzneimittel, das als Wirkstoff Hesperidin oder Hesperetin enthält, z.B. in Form von Tabletten, Kapseln, Pillen, Dragees, Granulaten, Suppositorien, Pellets, Lösungen, Dispersionen formuliert werden, wobei der Wirkstoff optional mit pharmazeutisch annehmbaren Hilfs- und Trägerstoffen kombiniert werden kann.

Liegt das erfindungsgemäße Arzneimittel in Form einer Lösung vor, so enthält diese bevorzugt 0.5 bis 20 Gew.-%, besonders bevorzugt 1 bis 10 Gew.-% des Wirkstoffs.

Die erfindungsgemäßen Arzneimittel, die Hesperidin oder Hesperetin als Wirkstoff enthalten, werden normalerweise gemäss herkömmlichen Methoden hergestellt und in einer pharmazeutisch geeigneten Form verabreicht.

Zum Beispiel können die festen oralen Formen zusammen mit dem Wirkstoff Streckstoffe, z.B. Lactose, Dextrose, Saccharose, Cellulose, Maisstärke oder Kartoffelstärke; Gleitmittel, z.B. Silikat, Talk, Stearinsäure, Magnesium- oder Calciumstearat und/oder Polyethylenglykole; Bindemittel, z.B. Stärken, Gummi arabicum, Gelatine, Methylcellulose, Carboxymethylcellulose oder Polyvinylpyrrolidon: Aufschussmittel, z.B. Stärke, Alginsäure, Alginate oder Natriumstärkeglykolate, aufschäumende Mischungen; Farbstoffe; Süßungsmittel; Benetzungsmittel, wie Lecithin, Polysorbate, Laurylsulfate: um im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen zur oralen Verabreichung können z.B. Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pectin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbaren Träger, z.B. steriles Wasser, Olivenöl, Ehyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonsichen Salzlösungen vorliegen.

Die Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Die Dosierungseinheit des Arzneimittels kann beispielsweise enthalten:
bei peroralen Arzneiformen:
   bevorzugt 800 bis 2000 mg, besonders bevorzugt 900 bis 1200 mg, Wirkstoff pro Tagesdosis.
   Die Tagesdosis kann beispielsweise in 1 bis 3 Einzeldosen, vorzugsweise in zwei Einzeldosen, täglich verabreicht werden.
   bei parenteralen Arzneiformen (zum Beispiel intravenös, subkutan, intramuskulär):
   bevorzugt 200 bis 600 mg, besonders bevorzugt 300 mg, Wirkstoff pro Tagesdosis.
   Die Tagesdosis kann beispielsweise in 1 bis 3 Einzeldosen, vorzugsweise in einer Einzeldosis, täglich verabreicht werden.
   bei Arzneiformen zur rektalen Applikation:
      bevorzugt 400 bis 800 mg, besonders bevorzugt 600 mg, Wirkstoff pro Tagesdosis.
      Die Tagesdosis kann beispielsweise in 1 bis 3 Einzeldosen, vorzugsweise in einer Einzeldosis, täglich verabreicht werden.
      bei Arzneiformen zur Applikation auf die Haut und Schleimhäute (z.B. Lösungen, Lotionen, Emulsionen, Salben usw.):
         bevorzugt 400 bis 800 mg Wirkstoff, besonders bevorzugt 600 mg, Wirkstoff pro Einzeldosis.
         Die Tagesdosis kann beispielsweise in 1 bis 6 Einzeldosen, vorzugsweise in 1 -3 Einzeldosen, täglich verabreicht werden.
         als Nahrungsergänzungsmittel:
            bevorzugt 200 bis 600 mg Wirkstoff, besonders bevorzugt 400 mg als Zugabe zu Getränken.

Bei Verwendung eines pharmazeutisch annehmbaren Salzes muss die verwendete Menge in dem Fachmann bekannter Weise entsprechend angepaßt werden.

### Testverfahren 1 (Tele-Stereo-EEG)

Die Veränderungen der EEG-Frequenzen nach intraperitonealer Gabe von Salzlösung (Kontrolle) bzw. verschiedener Dosen von Hesperidin (10 bis 300 mg/kg Körpergewicht sowie oral 500 mg/kg) wurden bestimmt. Ebenso wurden verschiedene Dosierungen von Hesperetin (80-320 mg/kg i.p. sowie 500 mg/kg oral) untersucht.

(0029) Die Untersuchungen wurden analog der durch W. Dimpfel beschriebenen Methode (Dimpfel W Preclinical data base of pharmaco-specific rat EEG fingerprints (Tele-Stereo-EEG). Eur J Med Res (2003) 8: 199-207) folgendermaßen durchgeführt:

Zwölf männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-Fourier-Transformation unterworfen und die Leistungsdichtespektren jeweils über 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Substanzen: die Substanzen wurden i.p. 45 Minuten nach Beginn der Messungen (Vorwert) injiziert. Fünf Minuten später wurden die Messungen wieder gestartet, mindestens über die nächsten 5 Stunden kontinuierlich analysiert und in 60-minütigen Perioden zusammengefaßt. Die Testsubstanzen wurden in einer Dosierung von 10 bis 500 mg/kg appliziert. Die experimentelle Serie wurde mit der Injektion von Salzlösung (Kontrolle), die zu keiner auffälligen Änderung führte, begonnen.

Der statistische Vergleich der Versuche zu den Ergebnissen, die nach Gabe von Salzlösung gemessen wurde, erfolgte mit Hilfe einer multivariaten Analyse nach Ahrens und Läuter (siehe H. Ahrens, J. Läuter "Mehrdimensionale Varianzanalyse" (1974), Akademie Verlag, Berlin) auf der Basis der Veränderungen innerhalb der einzelnen Frequenzbänder in allen Hirnregionen als Variablen.

Die Verabreichung von Salzlösung führte kaum zu Veränderungen der elektrischen Aktivität (µV²/Ω) im Vergleich zu den Vorphasenwerten.

Die Verabreichung von Hesperidin führte dosis-abhängig zu deutlichen Veränderungen der Leistungsdichte in den einzelnen Hirnregionen (nicht gezeigt). Erste Veränderungen in Form von Zunahmen der alpha1 und beta Aktivität wurden nach Gabe von 20 mg/kg i.p. beobachtet. Auch bei Gabe von 40 mg/kg dominierten Zunahmen der elektrischen Leistung im alpha1 und beta Bereich. Nach Gabe von 80 und 160 mg/kg entwickelte sich ein statistisch signifikantes Muster, welches nunmehr auch Abnahmen der elektrischen Leistung im delta, theta und alpha2 Bereich erkennen ließ und über längere Zeit zu beobachten war. Dieses Muster wurde bei dieser Dosis über die gesamte Versuchszeit von 5 Stunden beobachtet. Eine nochmalige Steigerung der Dosis auf 300 mg/kg i.p. führte zu einem generellen Abfall der elektrischen Leistung. Die Veränderungen waren statistisch mindestens auf dem P < 5%-Level signifikant.

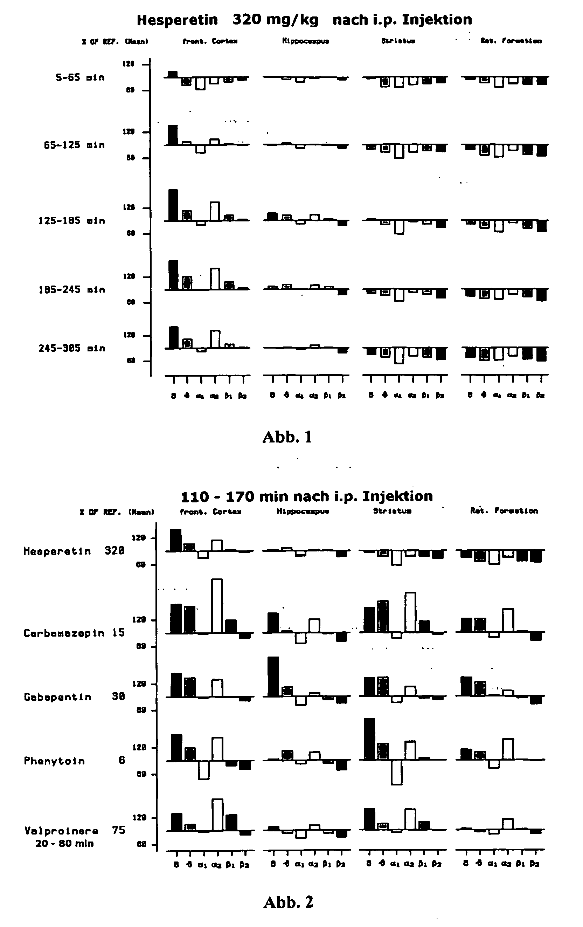

Die i.p. Verabreichung von Hesperetin als Einzeldosis führt zu Veränderungen der elektrischen Hirnaktivität bei den Testtieren, das im niedrigen Dosisbereich dem nach Gabe von Hesperidin entsprach. Dieses Muster korreliert in signifikanter Weise mit den Mustern, die für Medikamente, wie sie bereits für die Migräneprophylaxe (Metoprolol) und Schizophrenie (Risperidon) üblicherweise verwendet werden, nicht jedoch mit Mustern, die bei Medikamenten für andere Indikationen auftreten. Insbesondere bei höherer Dosierung von 320 mg/kg wurden nach Ablauf von 2 Stunden im frontalen Kortex Zunahmen der delta und alpha2 Aktivität beobachtet (siehe Abb. 1), wie sie typischerweise für die Applikation von Antikonvulsiva zu diesem Zeitpunkt gemessen wurden (siehe Abb. 2).

Die beobachteten Frequenzänderungen wurden mittels einer Diskriminanzanalyse mit den Ergebnissen von Medikamenten zur Behandlung von neurologischen und psychiatrischen Erkrankungen des Gehirns wie Valproinsäure (Epilepsie) verglichen (Abb. 3). Folgende Arzneimittel, welche bereits im selben Modell geprüft wurden, standen für Vergleichszwecke zur Verfügung (siehe Abb. 3) : Memantine 1.0 mg; Haloperidol 0.5 mg; Chlorpromazine 0.5 mg; Prothipendyl 1 mg; Clozapine 3 mg; Thioridazine 5 mg; Phentytoin 4 mg; Carbamazepine 15 mg; Valproinsäure 75 mg; Diazepam 0.5 mg; Methohexital 20 mg; Midazolam 0.5 mg; Meprobamat 60 mg; Phenobarbital 60 mg; Amphetamin 0.2 mg; Fenfluramin 1 mg; Kokain 10 mg; Mescaline 10 mg; Metanicotine 1mg; R-DOI 0.1 mg; R-DOM 0.2 mg; Dizocilpine 0.25 mg; LSD 0.05 mg; Acetylsalicylsäure 200 mg; Metamizol 100mg; Fentanyl 0.075 mg; Tramadol 10mg; Fluvoxamin 40 mg; Nomifensin 1mg; Mianserin 5 mg; Amitriptylin 10mg; Imipramin 10 mg; Die Angaben beziehen sich jeweils auf eine Dosierung in mg/kg Körpergewicht.

### Testverfahren 2 (Langzeitpotenzierung im Hippokampus-Schnittpräparat in vitro)

Wirkungen von Hesperidin und Hesperetin auf die Erregbarkeit der Pyramidenzellen wurden im Hippokampus-Schnittpräparat der Ratte bestimmt. Die Analyse der Wirkungen erfolgte auf drei Ebenen. Zunächst wurde anhand der Applikation von Einzelreizen die normale Erregbarkeit überprüft und anschließend nach einer sog. "Theta-burst-Stimulation" die Auswirkungen auf die Langzeitpotenzierung gemessen. Im nächsten Schritt wurde versucht, die Wirkung von Krampf auslösenden Substanzen zu antagonisieren. In einer dritten Versuchsserie wurde die Substanz eine Woche lang appliziert und dann "ex vivo" die Änderung der Erregbarkeit auf eine Provokation mit der Krampf auslösenden Substanz 3-Aminopyridin (3-AP) gemessen.

Für die Durchführung dieser Studien wurden 30 erwachsene männliche CD-Ratten verwendet. Die Isolation des Hippokampus erfolgte an äthemarkotisierten und anschließend exsanguinierten Tieren. In Phosphat-gepufferter Salzlösung (NaCl: 124 mM; KCI: 5 mM; CaCl: 2 mM; MgSO4: 2 mM; NaH2PO4: 1,25 mM; NaHCO3: 26 mM: Glucose: 10 mM; Kontroll-Lösung: Artifizielle Cerebral-Spinal-FLüsssigkeit (ACSF); alle Substanzen stammen von der Firma Roth, Karlsruhe, DE. Dann wurde der mittlere Teil des Hippokampus mit Hilfe eines Schnellklebers aufgeblockt und mit einem Vibratom in 400 µm dicke Scheiben geschnitten. Die Aufbewahrung dieser Schnitte erfolgte für mindestens eine Stunde vor Versuchsbeginn (siehe S.J. Schiff, G.G. Somjen "The effects of temperature on synaptic transmission in hippocampal tissue slices" Brain Research (1985), 345: 279-284) in einer mit Carbogen durchperlten Inkubationskammer.

Das Experiment selbst wurde in einer sog. "Base Unit" mit Haas Top" (Firma Medical Systems Corp., USA) bei 35 Grad Celsius durchgeführt. Der mit Hilfe peristaltischer Pumpen (Infusomat B. Braun Melsungen AG) superfundierte Hippokampus-Schnitt lag auf einem Stück Gaze. Eingeleitetes Carbogen hielt die erforderliche Sauerstoffzufuhr der Lösung aufrecht. Die Durchflussgeschwindigkeit betrug 200 ml/h.

Die Stimulation der CA2-Region (Schaffer-Kollateralen) erfolgte unter Verwendung eines Reizgenerators (Laborcomputer Labteam der Fa. MediSyst GmbH, Linden, DE) über eine Isoliereinheit mit Hilfe einer bipolar konzentrischen Stahlelektrode (Rhodes Medical Systems, USA. Die Impulsbreite betrug 200 µs, die Stromstärke konstant 200 µA. Der Reizgenerator löste im Abstand von jeweils 20 Sekunden 4 Einzelreizungen aus, die im Schnitt insgesamt 4 Populationsspikes evozierten. Die Ableitung der Antwort erfolgte in der CA3 Region. Das System mittelte die Reizantwort der 4 Spike-Amplituden.

Die Wirkung der beiden einzelnen Substanzen auf das evozierte Potential wurde nach Einzelreizung wie auch nach Induktion der Langzeitpotenzierung durch einen kurzdauernden (1 s) tetanischen Reiz (90 Hz) untersucht (siehe K.G. Reymann, H.K. Matthies, U. Frey, V.S. Voborbyev, H. Matthies "Calcium-induced long-term potentiation in the hippocampal slice. Characterization of the time course and conditions", Brain Bulletin (1986), 17: 291-296). Dabei wurde der Schnitt zunächst mit Kontrolllösung superfundiert. Nach Auffinden eines geeigneten Signals und Registrierung dieses Ausgangsignals während mehrerer Zeitpunkte wurde anstelle der Kontrolllösung auf Hesperidin- oder Hesperetin-haltige Lösung umgeschaltet. Jeder Schnitt wurde jeweils nur zu einem Experiment benutzt. Werte werden für n=6 Schnitte angegeben.

Die Ableitung der evozierten Antwort erfolgte in 10-minütigem Abstand extrazellulär mit einer gezogenen Glaskapillare (Elektrodenpuller, Rhema Labortechnik, DE). Das Antwortsignal wurde verstärkt (Verstärker "LMI", List Electronics, Darmstadt, DE) mit einem Laborrechnersystem Labteam (Software NeuroTool, MediSyst GmbH, Linden DE) analog digital gewandelt (Auflösung 12 Bit) und ausgewertet. Nach Auffinden eines geeigneten Signals (Amplitudenhöhe etwa 1 mV) wurde die Amplitudenhöhe des Populationsspikes (SAP=Summenaktionspotential; Popspike) als Ausgangsgröße festgelegt. Dieser Referenzwert ergab sich aus dem Mittelwert der letzten drei gemessenen Amplitudenhöhen während Superfusion mit der ACSF Lösung (Ausgangswert). Danach erfolgte die Superfusion mit Testsubstanz mit gleichem Procedere. In Folgeexperimenten erfolgte eine kurzfristige tetanische Reizung (Theta-Burst-Stimulus=TBS) zur Induktion der Langzeitpotenzierung. Hieraus resultierende Amplitudenveränderungen wurden in % dieses Ausgangswertes (n=6) angegeben. Werte in Gegenwart der Testsubstanz sind in % Reduktion dieses Referenzwertes angegeben.

Desweiteren wurde anstelle der Auslösung der Langzeitpotenzierung die Erregbarkeit der Pyramidenzellen in der Ausgangslage durch Superfusion mit einem Krampfgift, dem 4-Aminopyridin, verändert. In der Folge wurden dann gleichzeitig verschiedene Konzentrationen von Hesperidin oder Hesperetin mit 4-Aminopyridin zusammen superfundiert, um eine antagonistische Wirkung zu erkennen.

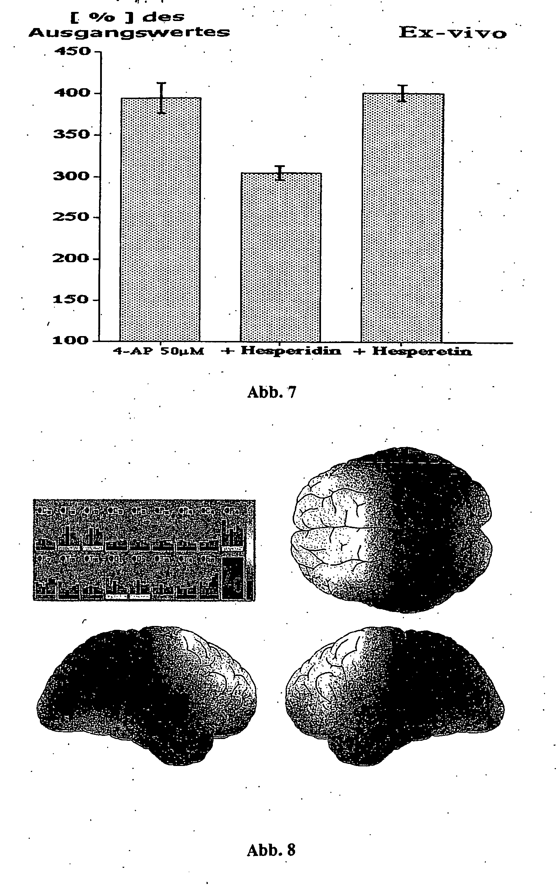

Die Ergebnisse dieser Untersuchungen sind in den Abb. 4 bis 7 gezeigt. Die erste Versuchsanordnung zeigt einen Konzentrations-abhängigen Abfall der Amplitude des Populationsspikes in Gegenwart von 5 bis 60 µM Hesperidin bei Einzelreizung (siehe Abb. 4). Die Auslösung der Langzeitpotenzierung durch TBS wurde bei der gleichen Konzentration Konzentrations-abhängig unterdrückt (siehe Abb. 5), wie es von Versuchen mit Antikonvulsiva her bekannt ist. Das Ergebnis ist in Abb. 6 dargestellt zur Velidierung der Versuchsbedingungen wurde des Ergebnis unter Anwendung des Antidepressivums Paroxetin bestätigt. Auch Paroxetin unterdrückt die Langzeitpotenzierung. Die Änderung der Erregbarkeit der Pyramidenzellen durch die Gegenwart von 4-Aminopyridin wurde von Hesperidin, nicht jedoch von Hesperetin vermindert (siehe Abb. 6). Aus diesem Ergebnis kann geschlossen werden, dass nicht nur Hesperetin - wie aus den EEG-Untersuchungen ersichtlich ist - sondern auch Hesperidin bestimmte Formen der Epilepsie positiv beeinflussen kann.

Dieselbe Versuchsanordnung wurde letztendlich noch einmal nach in vivo Applikation beider Substanzen in einer repetitiven Dosierung von 500 mg/kg täglich über 7 Tage im Vergleich zu Placebo angewandt (sog. "ex vivo" Design). Dabei sollte überprüft werden, ob eine repetitive Gabe zur Erregbarkeitsveränderungen der Pyramidenzellen des Hippokampus führt. Die Messung und Analyse der Empfindlichkeitsänderung ergab eine Unterdrückung der durch 4-Aminopyridin provozierten Erhöhung in den mit Hesperidin behandelten Tieren, nicht jedoch in den mit Placebo oder Hesperetin behandelten Tieren (siehe Abb. 7). Dieses Ergebnis ist auch ein Hinweis darauf, dass in vivo neben dem im Darm entstehenden Hesperetin auch genügend wirksames Hesperidin das Gehirn erreicht. Gleichzeitig wird dadurch nochmals bestätigt, dass Hesperidin und Hesperetin ein unterschiedliches Wirkprofil haben.

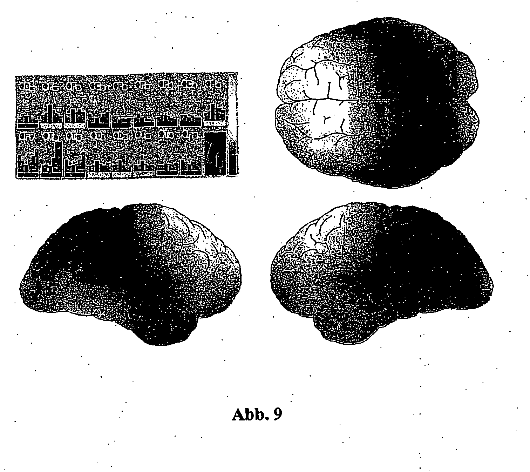

Um das Konzept auch am Patienten zu überprüfen, wurde einer jungen unter autosomaler dominanter nocturner Frontallappenepilepsie leidenden Patientin, die selbst in Gegenwart von 3 Gramm unterschiedlicher Antiepileptika weiterkrampfte (im Mittel 20 Anfälle pro Monat) täglich 1 Gramm Hesperidin abends vor dem Einschlafen oral über einen Zeitraum von 2 Monaten verabreicht. In dieser Zeit konnte die Häufigkeit um die Hälfte reduziert werden und die Nachuntersuchung ergab eine deutlich geringere Ausprägung des alphal Fokus im Frontallappen. Das Ergebnis der Untersuchungen ist in Abb. 8 und Abb. 9 dokumentiert.

### Kurze Beschreibung der Abbildungen

Abb. 1 Zeit-abhängige Wirkung von Hesperetin nach einer hohen Dosis von 320 mg/kg. Man beachte, dass nahezu nur der frontale Kortex von der Wirkung betroffen ist. Dies steht im Einklang mit der guten Bekömmlichkeit der Substanz.
Abb. 2 Vergleich der Wirkung von Hesperetin mit der nach Gabe von Antikonvulsiva.
Abb. 3 Dokumentation des Ergebnisses einer Diskriminanzanalyse auf der Basis von vier Hirnregionen und 6 Frequenzbändern (24 Variable). Die niedrige Dosierung von Hesperidin und Hesperetin ordnet sich in die Nachbarschaft von Metoprolol und von Neuroleptika, während eine hohe Dosierung von Hesperidin sich unter den Antidepressiva wiederfindet. Die hohe Dosierung von Hesperetin findet sich näher an den Antikonvulsiva. Die Zahlen hinter dem Präparat geben die Dosierung in mg/kg an.
Abb. 4 Konzentrations-abhängige Unterdrückung der Amplitude des Populationsspikes (Aktivität der Pyramidenzellen) in Gegenwart von Hesperidin im Hippokampus Schnittpräparat in vitro. Mittelwerte von je 6 Schnitten mit mittlerem Fehler des Mittelwertes sind dokumentiert.
Abb. 5 Verhalten der Amplituden des Populationsspikes im Hippokampus Schnittpräparat nach Theta-Burst-Stimulation in Gegenwart von Hesperidin wie sie auch von Antidepressiva her bekannt ist.
Abb. 6 Konzentrations-abhängige Unterdrückung des Effektes des Krampfgiftes 4-Aminopyridin auf die Amplitude des Populationsspikes im Hippokampus Schnittpräparat nach Einzelreizung durch Hesperidin, nicht jedoch durch Hesperetin. Die beiden Substanzen unterscheiden sich sehr deutlich.
Abb. 7 Ergebnisse im Hippokampus Schnittpräparat nach Applikation von Hesperidin oder Hesperetin in einer repetitiven Dosierung von 500 mg/kg täglich über 7 Tage in Vergleich zu Placebo. Die Erregbarkeit - gemessen in Gegenwart des Krampfgiftes 4-Aminopyridin - ist deutlich geringer nach Gabe von Hesperidin. Hesperetin hat keinen Effekt!
Abb. 8 und 9 Vergleich der elektrischen Hirnaktivität (EEG) einer Epilepsiepatientin (Alter: 20 Jahre, Diagnose: autosomale dominante nocturne Frontallappenepilepsie) vor und nach zusätzlicher zweimonatiger Gabe von 1 Gramm Hesperidin oral täglich vor dem Schlafengehen. Eine deutliche Verminderung des alpha1 Herdes frontal (Elektrodenpositionen F3, F4 und Fz ist erkennbar. Die alpha1 Aktivität ist in Form der Schwarzkörperstrahlung dargestellt (je heller desto höhere elektrische Leistung). Zudem konnte die Anzahl der Krämpfe halbiert werden. Abb. 8: vor Beginn der zusätzlichen Therapie, Abb.9: 2 Monate nach Beginn der Therapie.

## Patentansprüche

1. Verwendung von Hesperidin (bzw. seinem Aglykon Hesperetin) oder seinen pharmazeutisch annehmbaren Salzen zur Herstellung eines Arzneimittels oder Nahrungsergänzungsmittels zur Prophylaxe bzw. Behandlung von neurologischen und psychiatrischen Erkrenkungen des zentralen Nervensystems mit pathophysiologischen Erregbarkeitsstörungen, wobei die Erkrenkung ist Epilepsie.

2. Verwendung nach Anspruch 1, **dadurch gekennzeichnet, dass** das Nahrungsergänzungs- oder Arzneimittel in einer Dosierung von 400 bis 3000 mg, insbesondere von 800 bis 1800, bevorzugt von 1200 bis 1500 mg pro Tag zu verabreichen ist.

3. Verwendung nach Anspruch 1 oder 2, **dadurch gekennzeichnet, dass** das Nahrungsergänzungs- oder Arzneimittel zusätzlich pharmazeutisch annehmbare Träger, Hilfs- und/oder Verdünnungsmittel enthält.

4. Verwendung nach einem der Ansprüche 1,2 oder 3, **dadurch gekennzeichnet, dass** das Nahrungsergänzungs- oder Arzneimittel zur oralen Verabreichung hergerichtet wird.

## Claims

1. Use of hesperidine (or its aglycone hesperetine) or pharmaceutically acceptable salts thereof for the manufacture of a medicament or dietary supplement for prophylaxis or treatment of neurological and psychiatric diseases of the central nervous system with pathophysiological excitability disorders, wherein the disease is epilepsy.

2. The use according to claim 1, **characterized in that** the dietary supplement or medicament must be administered in a dosage from 400 to 3000 mg, particularly from 800 to 1800, preferably from 1200 to 1500 mg per day.

3. The use according to claim 1 or 2, **characterized in that** the dietary supplement or medicament further comprises pharmaceutically acceptable carriers, additives and/or diluents.

4. The use according to one of claims 1, 2 or 3, **characterized in that** the dietary supplement or medicament is made for oral administration.

## Revendications

1. Utilisation de l'hespéridine (et/ou de son hespérétine d'aglycone) ou d'un de ses sels pharmaceutiquement acceptables pour la fabrication d'un médicament ou d'un complément alimentaire afin de traiter la prophylaxie et/ou des maladies neurologiques ou psychiatriques du système central nerveux accompagnées de perturbations d'excitabilité pathologiques, dont la maladie est l'épilepsie.

2. Utilisation selon la revendication 1, **caractérisée en ce que** le complément alimentaire ou le médicament est administré à une dose de 400 à 3 000 mg, en particulier de 800 à 1 800, et de manière encore plus particulière de 1 200 à 1 500 mg par jour.

3. Utilisation selon la revendication 1 ou 2, **caractérisée en ce que** le complément alimentaire ou le médicament contient en outre un solvant, un adjuvant et/ou un diluant pharmaceutiquement acceptable.

4. Utilisation selon l'une des revendications 1, 2 ou 3, **caractérisée en ce que** le complément alimentaire ou le médicament est préparé pour une administration par voie orale.
